## Europäisches Patentamt
## European Patent Office
## Office européen des brevets

(19)

(11) Numéro de publication: **0 225 819**
**B1**

(12)

# FASCICULE DE BREVET EUROPEEN

(45) Date de publication du fascicule du brevet:
**10.01.90**

(51) Int. Cl.⁴: **A61F 2/34**

(21) Numéro de dépôt: **86402359.3**

(22) Date de dépôt: **22.10.86**

(54) **Composant cotyloïdien pour prothèse de hanche non cimentée.**

(30) Priorité: **25.10.85 FR 8515844**

(43) Date de publication de la demande:
**16.06.87 Bulletin 87/25**

(45) Mention de la délivrance du brevet:
**10.01.90 Bulletin 90/2**

(84) Etats contractants désignés:
**AT BE CH DE ES GB IT LI LU NL SE**

(56) Documents cités:
**EP-A- 0 121 002**
**DE-A- 2 822 585**
**FR-A- 2 416 004**
**US-A- 3 608 096**
**US-A- 3 840 904**

(73) Titulaire: **Duthoit, Etienne, 8 allée des Hêtres Louvil,**
**F-59830 Cysoing(FR)**
Titulaire: **Epinette, Jean-Alain, 27 rue Lamandin,**
**F-62700 Bruay-en-Artois(FR)**
Titulaire: **Carlier, Yves, 13 rue Pierre Lhermitte,**
**F-80000 Amiens(FR)**

(72) Inventeur: **Duthoit, Etienne, 8 allée des Hêtres Louvil,**
**F-59830 Cysoing(FR)**
Inventeur: **Epinette, Jean-Alain, 27 rue Lamandin,**
**F-62700 Bruay-en-Artois(FR)**
Inventeur: **Carlier, Yves, 13 rue Pierre Lhermitte,**
**F-80000 Amiens(FR)**

(74) Mandataire: **CABINET BONNET-THIRION, 95 Boulevard**
**Beaumarchais, F-75003 Paris(FR)**

**Description**

L'invention a trait à des prothèse de hanche et plus particulièrement à un composant cotyloïdien de prothèse de hanche, destiné à être implanté sans ciment et comprenant une coupole métallique à fixer dans la cavité cotyloïde convenablement préparée, avec un axe polaire orienté sensiblement dans la direction moyenne du col du fémur, et une cupule en matériau polymère adaptée à s'emboîter exactement dans la coupole pour former garniture de frottement et accueillir la tête sphérique d'un composant fémoral en sorte de reproduire l'articulation naturelle.

A l'origine les composants de prothèse, fémoraux et cotyloïdiens étaient fixés respectivement dans le fémur et dans le cotyle de l'os iliaque, à l'aide d'un ciment polymérisable, généralement de type acrylique.

Cette technique présente quelques inconvénients avec notamment la création, dans les os intéressés, de lignes de forces orientées suivant des directions non naturelles, source de douleurs, et la perte de plasticité du scellement avec le temps, avec formation de jeu entre l'os et le composant correspondant.

On a alors réalisé des composants de prothèse de hanche aptes à être implantés sans ciment, L'ancrage dans les os qui les reçoivent est assuré par des liaisons mécaniques résistantes.

Les composants cotyloïdiens, auxquels se rapporte la présente invention, se classent, dans l'état de la technique, en trois catégories.

Dans une première catégorie le composant comporte une calotte ou coupole métallique en anneau, munie d'un filetage extérieur périphérique qui doit prendre dans un taraudage complémentaire pratiqué dans la paroi du cotyle. Les filetages sont fréquemment autotaraudants, avec des filets interrompus à arêtes tranchantes.

La mise en place du composant en précontrainte doit assurer l'irréversibilité du vissage. Toutefois il se produit parfois une ostéolyse du cotyle, avec prise de jeu du composant. En outre le trajet des lignes de force depuis le fémur jusq'à l'os iliaque est quelque peu différent de ce qu'il est dans l'articulation naturelle, saine, avec une obliquité par rapport aux directions de plus grande résistance naturelle de la charpente osseuse.

Dans une seconde catégorie le composant comporte une calotte ou coupole sensiblement hémisphérique dont la surface extérieure est garnie d'un revêtement de métal poreux (voir par exemple US-A 3 840 904). Ce type de composant, prévu à l'origine pour améliorer une liaison entre le composant et un ciment, s'est montré apte à favoriser une liaison directe entre le composant métallique et l'os spongieux en croissance qui s'infiltre dans les porosités du métal.

En fait le mécanisme de liaison par croissance de l'os spongieux qui s'infiltre dans les porosités du métal, dit couramment "réhabitation" n'est efficace qu'après un laps de temps qui se compte en semaines. Aussi est-il prévu un ancrage primaire, immédiat, obtenu par des vis qui traversent des passages dans la calotte ou coupole et sont engagées dans des trous taraudés pratiqués dans la paroi du cotyle. Toutefois l'ancrage primaire est relativement fragile, surtout lorsque le sujet présente un vieillissement des os.

Les deux premières catégories de composants cotyloïdiens ont l'inconvénient important de ne pas respecter une des mécaniques fondamentales dans le fait que les déformations du cotyle soumis aux contraintes de traction et de compression en réponse à la charge de la hanche ne sont pas suivies avec suffisamment d'exactitude par les coupoles métalliques.

Il a été utilisé une troisième catégorie de composants cotyloïdiens qui se réduisant à une cupule, de forme extérieure sensiblement hémisphérique dans le cas général, et exécutée en matériau polymère, qui présente une élasticité voisine de celle de l'os. Plus exactement la souplesse du polymère lui permet de suivre les flexions élastiques de l'os où il est logé. L'ancrage primaire est assuré par un ou plusieurs tétons qui s'engagent dans des logements pratiqués dans le cotyle.

Malgré un comportement mécanique très proche de celui de l'os naturel, cette catégorie de composant est souvent réputée présenter des inconvénients, à savoir l'absence d'ancrage secondaire par formation d'une interface fibreuse entre l'os et le polymère, et des relargages du polymère (plastifiants) au contact direct des tissus, qui posent de graves problèmes de biocompatibilité.

Quoi qu'il en soit, l'invention a pour objectif un composant cotyloïdien dérivé de la seconde catégorie présentée ci-dessus qui satisfasse au mieux aux critères suivants :
- respect de la distribution des lignes de forces naturelles dans l'articulation saine et les os voisins;
- fixation primaire du composant dans l'os résistante et sûre ;
- contact précis avec l'os, qui, conjointement avec des propriétés biomécaniques proches de celles des os, permet un ancrage secondaire excellent par réhabitation osseuse.

A cet effet l'invention propose un composant cotyloïdien de prothèse de hanche destiné à être implanté sans ciment et comprenant une coupole métallique à fixer dans la cavité cotyloïde convenablement préparée, avec un axe polaire orienté sensiblement dans la direction moyenne du col du fémur, et une cupule en matériau polymère adaptée à s'emboîter exactement dans la coupole pour former garniture de frottement et accueillir la tête sphérique d'un composant fémoral en sorte de reproduire l'articulation naturelle, la surface extérieure de la coupole étant au moins partiellement garnie d'un revêtement de métal poreux apte à être envahi par de l'os spongieux en croissance, et des passages étant pratiqués dans la coupole pour des moyens d'ancrage pénétrant dans la paroi de la cavité cotyloïde, caractérisé en ce que la coupole, globalement hémisphérique avec un bord équatorial, présente sur sa surface externe une première zone occupant une calotte sphérique et garnie du revêtement de métal poreux, et une seconde zone restant généralement lisse, la première zone ayant un axe polaire angulairement décalé par rapport à celui de la coupole pour être orienté sensiblement suivant la direction naturelle de transmission de force du fémur à l'os iliaque et comportant les passages des moyens d'ancrage, tandis que la seconde zone comporte des fentes s'étendant suivant des méridiens de la coupole depuis le bord équatorial de celle-ci jusqu'à la limite avec la première zone.

La première zone, en forme de calotte sphérique, va se trouver étroitement appliquée contre le toit du cotyle, centrée sur l'axe moyen des lignes de force naturelles, de sorte que la liaison secondaire par "réhabilation" du revêtement de métal poreux va travailler en compression essentiellement. Par ailleurs les fentes de la seconde zone, conjointement avec la surface lisse de celle-ci, procurent au composant une souplesse qui permettra de suivre les déformations élastiques des cornes antérieure et postérieure du cotyle.

On remarquera que les axes polaires de la coupole hémisphérique et de la calotte sphérique qui constitue la première zone sont nécessairement concourants et se coupent au centre de la sphère génératrice de la coupole hémisphérique et de la première zone. En raison de l'angle que forment ces deux axes polaires, ils définissent un plan qui sera dit plan commun des axes polaires. Une conséquence directe du décalage angulaire des axes polaires de la coupole hémisphérique et de la calotte sphérique qui forme la première zone, est que la seconde zone, qui occupe le reste de l'hémisphère est de largeur variable, prise suivant les méridiens de la coupole, la largeur de cette seconde zone passant par les extremuns dans le plan commun des axes polaires, c'est-à-dire étant minimale dans ce plan du même côté que l'axe polaire de calotte par rapport à l'axe polaire de coupole, et maximale du côté opposé. Il en résulte que l'élasticité apportée à la coupole par les fentes de seconde zone sera maximale dans la région des cornes du cotyle.

D'ailleurs, de préférence, la calotte sphérique constituant la seconde zone a un bord tangent au bord équatorial de la coupole, la largeur minimale de la seconde zone étant nulle, ce qui permet de donner une élasticité importante à la partie de seconde zone qui doit venir se placer dans la région des cornes du cotyle tout en donnant à la première zone une surface suffisante pour la liaison avec le toit du cotyle par "réhabilation".

En disposition préférée les passages de moyens d'ancrage sont au nombre de deux, disposés symétriquement par rapport au plan commun des axes polaires. Cette disposition favorise la mise en position correcte du composant, et notamment l'orientation appropriée de l'axe polaire de la calotte de première zone.

De préférence en outre ces passages sont ménagés dans les axes de deux canons cylindriques en saillie sur la face externe de la coupole, ces axes étant parallèles entre eux et parallèles au plan commun des axes polaires. D'une part ces canons se placent dans des logements convenablement alésés dans le toit du cotyle, avec un jeu réduit, ce qui assure une mise en position précise du composant; d'autre part ces canons forment guides pour des vis formant moyens d'ancrage, ces vis pénétrant dans le toit du cotyle à une position et avec une orientation précise, déterminées pour qu'elles se placent dans une partie massive de l'os iliaque.

La surface extérieure des canons est de préférence garnie de métal poreux, pour favoriser un ancrage secondaire qui s'oppose à tout déplacement de la coupole par rapport au toit du cotyle, l'os spongieux de réhabilation travaillant essentiellement en compression.

En disposition préférée la coupole présente un logement de cupule avec un fond en calotte sphérique concave relié au bord équatorial par une paroi tronconique.

Cette disposition assure, avec un bon appui réparti de la cupule sur le fond de son logement dans la coupole, la coïncidence des axes polaires de la coupole et de la cupule en raison de l'emboîtement tronconique.

Il sera souvent avantageux que le logement de la tête sphérique du composant fémoral associé, ménagé dans la cupule, embrasse un angle sphérique un peu supérieur à l'hémisphère, afin que la tête sphérique du composant fémoral puisse pénétrer dans son logement sans effort excessif, mais soit maintenu dans ce logement de façon positive.

D'autres caractéristiques et avantages de l'invention ressortiront d'aileurs de la description qui va suivre, à titre d'exemple, en référence aux dessins annexés dans lesquels :

la figure 1 est une vue perspective d'un composant cotyloïdien selon l'invention ;
la figure 2 est une vue éclatée d'une prothèse de hanche incorporant un composant selon l'invention ;
la figure 3 est une élévation latérale d'un composant cotyloïdien ;
la figure 4 est une vue de dessus du composant cotyloïdien de la figure 3 ;
la figure 5 est une coupe suivant le plan V-V de la figure 4.

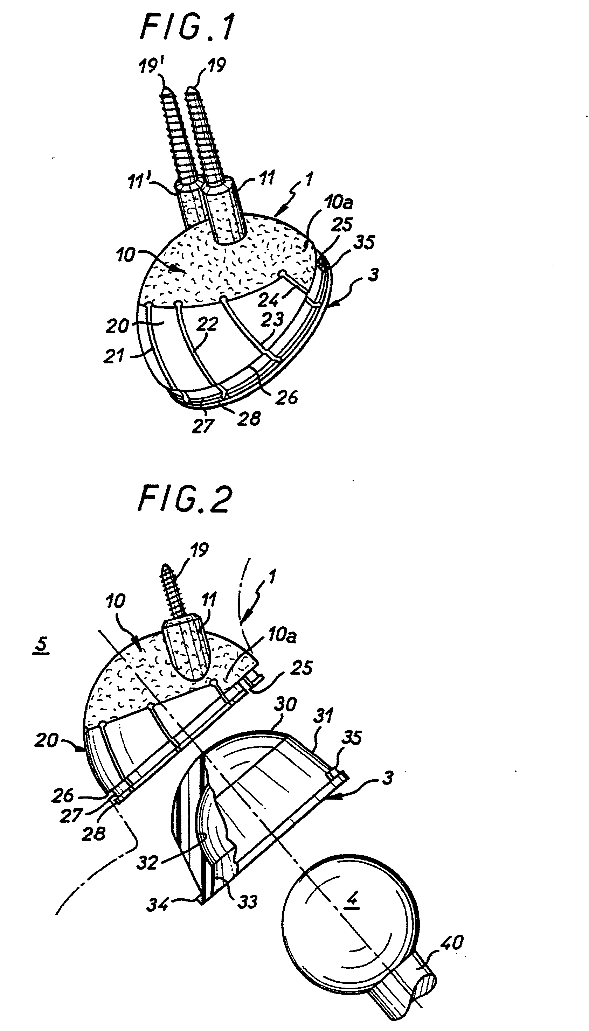

Selon la forme choisie et représentée sur les figures 1 à 5, le composant cotyloïdien classiquement comprend une coupole métallique 1 qui se fixe dans la cavité cotyloïde de l'os iliaque 5, et une cupule 3 en matériau polymère qui s'emboîte exactement dans la coupole 1, et est capable d'accueillir dans une cavité 32 la tête sphérique 4 d'un composant fémoral, tête fixée à l'extrémité d'un col 40. On notera que la coupole 1 et la cupule 3 présentent des axes polaires 1B confondus avec la direction moyenne de l'axe du col 40.

Selon l'invention la coupole métallique 1 en titane, de forme généralement hémisphérique, présente une première zone 10 en forme générale de calotte sphérique avec un axe polaire 10$\underline{b}$ qui forme avec l'axe polaire 1$\underline{b}$ de la coupole 1 un angle A1 d'environ 30°. La calotte sphérique 10 s'etend dans un plan méridien sur environ 120°, de sorte que le cercle limite de la calotte est tangent au bord équatorial 26 de la calotte 1 en un point 26$\underline{b}$, évidemment dan le plan commun aux axes polaires 1$\underline{b}$ et 10$\underline{b}$.

La face externe de la coupole 1 comporte une seconde zone 20, située entre la calotte sphérique 10 et le bord équatorial 26 de la coupole 1. Dans cette seconde zone 20 sont usinées des fentes 21,22,22',23,23',24,24' qui s'étendent sur toute la largeur de la seconde zone 20 suivant des méridiens de la coupole 1. La fente 21, la plus longue, part du point 26a du bord équatorial 26 situé dans le plan commun des axes polaires 1b et 10b, et diamétralement opposé au point 26b où la calotte sphérique 10 est tangente au bord équatorial 26.

Les fentes 22 et 22', 23 et 23', 24 et 24' sont respectivement symétriques par rapport au plan commun des axes polaires 1b et 10b. Les fentes extrêmes 24 et 24' sont espacées angulairement de 110° suivant le cercle équatorial de la coupole 1, et, entre ces fentes extrêmes 24 et 24', les fentes 23,22,21,22',23' sont régulièrement espacées angulairement suivant le cercle équatorial de la coupole 1. Les fentes se terminent au bord de la calotte sphérique 10 par des alésages normaux à la coupole, ces alésages étant prévus pour réduire la concentration des contraintes dues à la flexion des lobes de la seconde zone 20.

La première zone 10 se prolonge, au-delà de la calotte sphérique d'axe polaire 10b, jusqu'au bord équatorial 26 de la coupole entre les fentes extrêmes 24 et 24', par deux triangles curvilignes 10a et 10'a.

Par ailleurs la coupole se prolonge, au-delà du bord équatorial 26, par une couronne cylindrique comprenant une gorge 27 suivie d'un jonc 28, afin de permettre la préhension de la coupole lors de sa mise en place dans la cavité cotyloïde. Cette couronne cylindrique comporte au moins une échancrure 25, dont la fonction sera précisée plus loin.

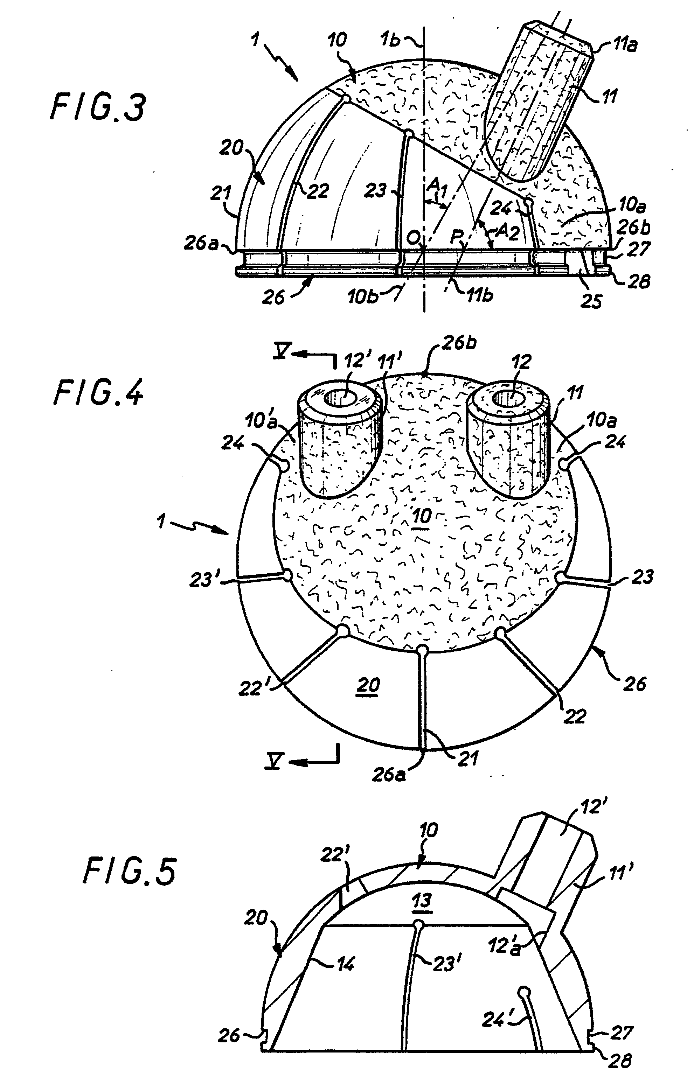

Dans la première zone 10 sont disposés deux canons 11 et 11', qui font saillie vers l'extérieur. Ces canons sont cylindriques et présentent des axes parallèles entre eux, parallèles au plan commun des axes polaires 1b et 10b de la coupole 1 et de la calotte 10. Les axes seront référencés 11b et 11'b; cet axe 11'b n'est pas représenté, se confondant sur la figure 3 avec l'axe 11b. D'ailleurs, dans ce qui suit, on désignera les éléments se rapportant aux canons 11 et 11' par une référence numérique, avec un indice prime pour le canon 11', même si les figures ne représentent que l'un de ces éléments, en raison de la symétrie des dispositions.

Les canons 11 et 11' sont percés d'alésages 12,12' suivant leurs axes 11b et 11'b, dans lesquels sont guidées des vis 19 et 19' (figures 1 et 2), ces vis 19 et 19' constituant ancrage primaire de la coupole 1 dans la cavité cotyloïdienne, en se vissant dans le toit du cotyle. La tranche supérieure des canons 11 et 11' comporte un chanfrein 11a, 11'a. A la base de ces canons, dans la paroi interne de la coupole 1 sont pratiqués des lamages 12a, 12'a où s'encastre la tête des vis 19, 19'.

La face externe de la première zone 10 en calotte sphérique, y compris les triangles curvilignes 10a et 10'a, les faces latérales et les tranches terminales des canons 11 et 11' sont garnies d'un revêtement de titane poreux, obtenu par projection à la torche à plasma, destiné à être envahi par de l'os spongieux en croissance pour former ancrage secondaire du composant cotyloïdien.

Les axes 11b et 11'b des canons 11 et 11' forment avec le plan équatorial de la coupole 1, où est situé le bord équatorial 26, un angle dièdre A2 compris entre 60 et 70° et de préférence 65°. L'arête P de ce dièdre, vue en bout figure 3, est à une distance du centre 0 du bord équatorial 26 de la coupole 1 qui est sensiblement égale à 2/9 du rayon de l'hémisphère de coupole.

Par ailleurs les canons 11 et 11' ont des diamètres de sensiblement 10 mm, et leurs axes 11b et 11'b sont écartés d'environ 20 mm. Toutes les dimensions indiquées ci-dessus ont été déterminées pour que les trajets des lignes de forces entre le fémur et l'os iliaque soient conformes en moyenne aux trajets naturels.

La forme intérieure de la coupole 1 est complémentaire de celle de la forme extérieure de la cupule 3. Elle comporte une calotte sphérique 13 concave de même centre que la coupole 1, et un tronc de cône 14, d'axes confondus avec l'axe polaire de la coupole 1. La cupule 3, en matériau polymère biologiquement compatible, comporte de façon correspondante, extérieurement, une calotte sphérique 30 et un tronc de cône 31. La coopération des troncs de cône 14 et 31 assure la coïncidence des axes polaires de la coupole 1 et de la cupule 3, tandis que l'appui mutuel des calottes 13 et 30 facilite la transmission des efforts

A l'intérieur de la cupule 3 est ménagée une cavité sensiblement hémisphérique 32 correspondant à la tête sphérique 4 du composant fémoral associé. On notera que la cavité 32 s'étendra avantageusement sur un peu plus d'une hémisphère pour que l'emboîtement de la tête sphérique 4 nécessite une légère déformation de l'ouverture de la cavité 32, et que cette tête 4 soit maintenue dans la cavité.

Le bord de la cavité 32 se prolonge par un chanfrein à 45° 33 qui facilite l'introduction de la tête 4 dans la cavité 32, et constitue, en coopération avec le col cylindrique 40 du composant fémoral, limitation du débattement de la prothèse.

Le tronc de cône 31 de la cupule 3 se termine par un rebord 34 annulaire qui vient en contact avec le jonc 28 de la coupole 1 lors de la mise en place de la cupule 3, et facilite les manipulations de cette cupule. A la base du tronc de cône 31 et en saillie du rebord 34 est aménagé au moins un téton 35 qui vient s'engager dans l'échancrure 25 de la couronne 27, 28 de la coupole 1, et bloque en rotation la cupule 3 dans la coupole 1.

Pour la mise en place de la prothèse, bien que cette opération en soi sorte du cadre de la présente invention, on prépare la cavité cotyloïde en forme hémisphérique, de diamètre convenable et, à l'aide de calibres on pratique les logements des tétons, avec une orientation telle que l'axe polaire 1b de la coupole 1 soit environ à 40° d'un plan perpendiculaire à l'axe général du tronc, avec le plan méridien de la fente centrale 21 vertical (par rapport au sujet debout). On met en place cette coupole 1, et on engage à fond les vis 19 et 19' dans le toit du cotyle. On met ensuite en place la cupule 3, et on peut reconstituer l'articulation en emboîtant dans la cavité intérieure de la cupule 3 la tête sphérique 4 d'un composant fémoral, qui entre-temps aura été engagé dans le fémur.

On appréciera que les tétons 11 et 11' s'opposent à toute rotation de la coupole 1 dans la cavité cotyloïde, dès le stade de l'ancrage primaire, de façon plus précise que par des vis seules. Ainsi l'envahissement du revêtement de titane poreux par l'os spongieux en croissance ne sera pas retardé ou gêné par des petits déplacements de la coupole par rapport au cotyle lorsque l'articulation fonctionne.

Par ailleurs le soudage de la coupole 1 à l'os iliaque 5, par "réhabilitation" se produit dans la première zone, correspondant au toit du cotyle où les flexions osseuses sont très faibles,tandis que la seconde zone 20 reste susceptible de faibles déplacements par rapport à l'os sur lequel elle s'appuie, de sorte que l'élasticité de cette seconde zone, résultant des fentes, peut accompagner les flexions osseuses, d'autant plus sensibles que l'on se rapproche des cornes antérieure et postérieure du cotyle. La latitude de déformation élastique de la seconde zone 20 va en effet croissant avec la largeur de cette zone.

Bien entendu l'invention n'est pas limitée aux exemples décrits, notamment lorsque la description fait état de grandeurs numériques, mais en embrasse toutes les variantes d'exécution dans la portée des revendications.

**Revendications**

1. Composant cotyloïdien de prothèse de hanche destiné à être implanté sans ciment et comprenant une coupole métallique (1) à fixer dans la cavité cotyloïde convenablement préparée, avec un axe polaire (1$\underline{b}$) orienté sensiblement dans la direction moyenne du col du fémur, et une cupule (3) en matériau polymère adaptée à s'emboîter exactement dans la coupole (1) pour former garniture de frottement et accueillir la tête sphérique (4) d'un composant fémoral en sorte de reproduire l'articulation naturelle, la surface extérieure de la coupole (1) étant au moins partiellement garnie d'un revêtement de métal poreux apte à être envahi par de l'os spongieux en croissance, et des passages (12,12') étant pratiqués dans la coupole (1) pour des moyens d'ancrage (19,19') pénétrant dans la paroi de la cavité cotyloïde, caractérisé en ce que la coupole (1), globalement hémisphérique avec un bord équatorial (26), présente sur sa surface externe une première zone (10) occupant une calotte sphérique et garnie du revêtement de métal poreux, et une seconde zone (20) restante généralement lisse, la première zone (10) ayant un axe polaire (10$\underline{b}$) angulairement décalé par rapport à celui (1$\underline{b}$) de la coupole (1) pour être orienté sensiblement suivant la direction naturelle de transmission de force du fémur à l'os iliaque et comportant les passages(12,12') des moyens d'ancrage (19,19'), tandis que la seconde zone (20) comporte des fentes (21-24,24') s'étendant suivant des méridiens de la coupole (1) depuis le bord équatorial (26) de celle-ci jusqu'à la limite avec la première zone (10).

2. Composant cotyloïdien selon la revendication 1, caractérisé en ce que la calotte sphérique (10) constituant première zone a un bord tangent au bord équatorial (26) de coupole (1).

3. Composant cotyloïdien selon la revendication 2, caractérisé en ce que les fentes (21,...24,24') sont en nombre impair, avec une fente centrale (21) dans le plan commun des axes polaires (1$\underline{b}$,10$\underline{b}$) de coupole (1) et de première zone (10), les autres fentes (22,23,24,22',23',24') étant disposées symétriquement par deux par rapport à ce plan/

4. Composant cotyloïdien selon la revendication 3, caractérisé en ce que les fentes (24,24') angulairement extrêmes par rapport à la fente centrale (21) sont écartées l'une de l'autre d'un angle d'environ 110°, la première zone s'étendant en outre entre ces fentes (24,24') extrêmes jusqu'au bord équatorial (26) de la coupole (1).

5. Composant cotyloïdien selon la revendication 4, caractérisé en ce que les fentes (21,...24,24') sont au nombre de sept, régulièrement espacées entre les fentes extrêmes (24,24').

6. Composant cotyloïdien selon une quelconque des revendications 1 à 5, caratérisé en ce que les passages (12,12') des moyens d'ancrage (19,19') sont au nombre de deux et disposés symétriquement par rapport au plan commun des axes polaires (1$\underline{b}$, 10$\underline{b}$) de coupole (1) et de première zone (10).

7. Composant cotyloïdien selon la revendication 6, caractérisé en ce que les passages (12,12') sont ménagés suivant les axes (11$\underline{b}$,11'$\underline{b}$) de canons cylindriques (11,11') en saillie sur la face externe de la coupole (1), ces axes (11$\underline{b}$,11'$\underline{b}$) étant prallèles entre eux et symétriques par rapport au plan commun des axes polaires (1$\underline{b}$, 10$\underline{b}$) de la coupole (11) et de première zone (10).

8. Composant cotyloïdien selon la revendication 7, caractérisé en ce que le plan défini par les axes (11$\underline{b}$,11'$\underline{b}$) des canons (11,11') fait un angle dièdre (A2) avec le plan du bord équatorial (26) de la coupole (1), compris entre 60 et 70°.

9. Composant cotyloïdien selon la revendication 8, caractérisé en ce que l'arête (P) de cet angle dièdre (A2) est à une distance du centre (O) du cercle de bord équatorial (26) égale à environ 2/9 du rayon de ce cercle.

10. Composant cotyloïdien selon une quelconque des revendications 7 à 9, caractérisé en ce que la surface extérieure des canons (11,11') est garnie d'un revêtement de métal poreux.

11. Composant cotyloïdien selon une quelconque des revendications 1 à 10, caractérisé en ce que le bord équatorial (26) présente un prolongement en couronne (27,28) où est pratiquée une gorge (27) s'ouvrant vers l'extérieur.

12. Composant cotyloïdien selon la revendication 11, caractérisé en ce que le prolongement en couronne (27,28) est entaillé par au moins une échancrure (25) où s'engage une saillie correspondante (35) de la cupule (3).

13. Composant cotyloïdien selon une quelconque des revendications 1 à 12, caractérisé en ce que la coupole (1) présente un logement de la cupule (3) avec un fond en calotte sphérique (13) relié au bord équatorial (26) par une paroi tronconique (14).

14. Composant cotyloïdien selon une quelconque des revendications 1 à 13, caractérisé en ce que la cupule (3) présente un logement (32) pour la tête sphérique (4) du composant fémoral qui s'étend sur plus d'une hémisphère, l'élasticité du bord extrême de la cupule étant suffisante pour laisser passer la tête sphérique (4).

## Patentansprüche

1. Gelenkpfannenelement einer Hüftgelenkprothese für zementfreie Implantation mit einer zur Befestigung in der entsprechend vorbereiteten Gelenkpfannenhöhlung dienenden Schale (1) aus Metall mit einer im wesentlichen in der mittleren Richtung des Oberschenkelhalses verlaufenden polaren Achse (1b) und mit einem Becher (3) aus Kunststoffmaterial, der genau in die Schale (1) paßt, die reibungsvermindernde Auskleidung bildet und den Kugelkopf (4) eines Oberschenkelelements aufnimmt, um die natürliche Gelenkfunktion nachzubilden, wobei die Außenfläche der Schale (1) mindestens teilweise mit einer porösen Metallbeschichtung versehen ist, in die das Knochengewebe einwachsen kann, und in der Schale (1) Kanäle (12, 12') für in die Wand der Gelenkpfannenhöhlung eindringende Verankerungsmittel (19, 19') ausgebildet sind, dadurch gekennzeichnet, daß die im ganzen halbkugelförmig mit einem äquatorialen Rand (26) ausgebildete Schale (1) auf ihrer Außenfläche eine erste Zone (10), die eine Kugelkalotte einnimmt und mit einer porösen Metallbeschichtung versehen ist, und eine restliche, im ganzen glatte zweite Zone (20) aufweist, wobei die erste Zone (10) eine polare Achse (10b), die einen Winkel mit der polaren Achse (1b) der Schale (1) bildet, so daß sie sich im wesentlichen in der natürlichen Richtung der Kraftübertragung vom Oberschenkel- zum Hüftknochen erstreckt, und die Kanäle (12, 12') der Verankerungsmittel aufweist, die sich längs Meridianen der Schale (1) von deren äquatorialen Rand (26) bis zur Grenze der ersten Zone (10) erstrecken.

2. Gelenkpfannenelement nach Anspruch 1, dadurch gekennzeichnet, daß die die erste Zone bildende Kugelkalotte (10) einen den äquatorialen Rand (26) der Schale (1) tangential berührenden Rand aufweist.

3. Gelenkpfannenelement nach Anspruch 2, dadurch gekennzeichnet, daß die Schlitze (21,...24, 24') in ungerader Zahl vorhanden sind, wobei ein Mittelschlitz (21) in der gemeinsamen Ebene der polaren Achsen (1b, 10b) der Schale (1) und der ersten Zone (10) liest und die anderen Schlitze (22, 23, 24, 22', 23', 24') paarweise symmetrisch zu dieser Ebene angeordnet sind.

4. Gelenkpfannenelement nach Anspruch 3, dadurch gekennzeichnet, daß die bezüglich des Mittelschlitzes (21) winkelmäßig äußersten Schlitze (24, 24') voneinander einen Winkelabstand von etwa 110° haben, wobei sich die erste Zone außerdem zwischen diesen beiden äußersten Schlitzen (24, 24') bis zum äquatorialen Rand der Schale (1) erstreckt.

5. Gelenkpfannenelement nach Anspruch 4, dadurch gekennzeichnet, daß die Zahl der Schlitze (21,...24, 24') sieben beträgt und diese in gleichmäßigen Abständen zwischen den äußersten Schlitzen (24, 24') verteilt sind.

6. Gelenkpfannenelement nach einem der Ansprüche 1 bis 5, dadurch gekennzeichnet, daß die Zahl der Kanäle (12, 12') der Verankerungsmittel (19, 19') zwei beträgt und daß die Kanäle symmetrisch bezüglich der gemeinsamen Ebene der polaren Achsen (1b, 10b) der Schale (1) und der ersten Zone (10) angeordnet sind.

7. Gelenkpfannenelement nach Anspruch 6, dadurch gekennzeichnet, daß die Kanäle (12, 12') gemäß den Achsen (11b, 11'b) zylindrischer Rohre (11, 11') ausgebildet sind, die von der Außenfläche der Schale (1) vorspringen, wobei diese Achsen zueinander parallel und symmetrisch zur gemeinsamen Ebene der polaren Achsen (1b, 10b) der Schale (1) und der ersten Zone (10) sind.

8. Gelenkpfannenelement nach Anspruch 7, dadurch gekennzeichnet, daß die von den Achsen (11b, 11'b) der Rohre (11, 11') definierte Ebene mit der Ebene des äquatorialen Randes (26) der Schale (1) einen zwischen 60 und 70° liegenden Diederwinkel (A2) bildet.

9. Gelenkpfannenelement nach Anspruch 8, dadurch gekennzeichnet, daß die Kante (P) dieses Diederwinkels (A2) vom Mittelpunkt (0) des Kreises des äquatorialen Randes (26) einen Abstand von etwa $2/9$ des Radius dieses Kreises hat.

10. Gelenkpfannenelement nach einem der Ansprüche 7 bis 9, dadurch gekennzeichnet, daß die Außenfläche der Rohre (11, 11') mit einer porösen Metallbeschichtung versehen ist.

11. Gelenkpfannenelement nach einem der Ansprüche 1 bis 10, dadurch gekennzeichnet, daß der äquatoriale Rand (26) eine kranzförmige Verlängerung (27, 28) aufweist, in der eine sich nach außen öffnende Nut (27) ausgebildet ist.

12. Gelenkpfannenelement nach Anspruch 11, dadurch gekennzeichnet, daß die kranzförmige Verlängerung (27, 28) mindestens eine eingeschnittene Ausnehmung (25) aufweist, in welche ein entsprechender Vorsprung (35) des Bechers (3) eingreift.

13. Gelenkpfannenelement nach einem der Ansprüche 1 bis 12, dadurch gekennzeichnet, daß die Schale (1) für den Becher (3) einen Sitz mit einem Boden in Form einer Kugelkalotte (13) aufweist, der mit dem äquatorialen Rand (26) durch eine kegelstumpfförmige Wand (14) verbunden ist.

14. Gelenkpfannenelement nach einem der Ansprüche 1 bis 13, dadurch gekennzeichnet, daß der Becher (3) ein Lager (32) für den Kugelkopf (4) des Oberschenkelelements aufweist, der sich über mehr als eine Halbkugel erstreckt, wobei die Elastizität des Außenrandes des Bechers ausreicht, um den Kugelkopf (4) hindurchtreten zu lassen.

## Claims

1. A cotyloid component of a hip prosthesis intended to be implanted without cement and comprising a metal dome (1) to be fixed in a suitably prepared cotyloid cavity, having a polar axis (1b) directed substantially in the medial direction of the femur neck, and a cup (3) of polymer material to be fitted precisely into the dome (1) so as to form a friction lining and to accommodate the spherical head (4) of a femoral component in such a way as to reproduce a natural joint, the outer surface of the dome (1) being at least partly lined with a covering of porous metal capable of being invaded by growing spongy tone, and passages (12, 12') being provided in the dome (1) for anchoring means (19, 19') which penetrate into the wall of the cotyloid cavity, characterised in that the dome (1), which is generally hemispherical with an equatorial edge (26), has or its outer surface a first zone (10) defining a spherical dome and lined with porous metal covering, and a generally smooth second zone (20), the first zone (10) having a polar axis (10b) which is angularly displaced in relation to the axis (1b) of the dome (1) so as to be directed substantially in the natural direction of transmission of force from the femur to the iliac bone, and comprising passages (12, 12') for the anchoring means (19, 19'), whereas the second zone (20) comprises slots (21–24, 24') extending along meridians of the dome (1) from the equatorial edge (26) thereof to the boundary with the first zone (10).

2. A cotyloid component according to claim 1, characterised in that the spherical dome (10) forming the first zone has an edge tangential to the equatorial edge (26) of the dome (1).

3. A cotyloid component according to claim 2, characterised in that the slots (21, ....24, 24') are odd in number, with a central slot (21) in the common plane of the polar axes (1b, 10b) of the dome (1) and the first zone (10), the other slots (22, 23, 24, 22', 23', 24') being arranged symmetrically in pairs relative to this plane.

4. A cotyloid component according to claim 3, characterised in that the slots (24, 24') angularly furthest from the central slot (21) are spaced apart from one another at an angle of about 110°, the first zone also extending between said furthest slots (24, 24') to the equatorial edge (26).

5. A cotyloid component according to claim 4, characterised in that the slots (21, ...24, 24') are seven in number and they are regularly spaced apart between the furthest slots (24, 24').

6. A cotyloid component according to any one of claims 1 to 5, characterised in that the passages (12, 12') of the anchoring means (19, 19') are two in number and they are disposed symmetrically relative to the common plane of the polar axes (1b, 10b) of the dome (1) and the first zone (10).

7. A cotyloid component according to claim 6, characterised in that the passages (12, 12') are provided along axes (11b, 11'b) of cylindrical projections (11, 11') protruding from the outer surface of the dome (1), these axes (11b, 11'b) being parallel to each other and symmetrical in relation to the common plane of the polar axes (1b, 10b) of the dome (1) and the first zone (10).

8. A cotyloid component according to claim 7, characterised in that the plane defined by the axes (11b, 11'b) of the projections (11, 11') forms a dihedral angle (A2) of between 60 and 70° with the plane of the equatorial edge (26) of the dome (1).

9. A cotyloid component according to claim 8, characterised in that the apex (P) of the dihedral angle (A2) is at a distance from the centre (0) of a circle of the equatorial edge (26), which is equal to about 2/9ths of the radius of said circle.

10. A cotyloid component according to any one of claims 7 to 9, characterised in that the outer surface of the projections (11, 11') is lined with a covering of porous metal.

11. A cotyloid component according to any one of claims 1 to 10, characterised in that the equatorial edge (26) has an annular extension (27, 28) in which an outwardly opening groove (27) is formed.

12. A cotyloid component according to claim 11, characterised in that the annular extension (27, 28) is provided with at least one recess (25) in which a corresponding projection (35) of the cup (3) engages.

13. A cotyloid component according to any one of claims 1 to 12, characterised, in that the dome (1) has a cavity of the cup (3) with a spherical domed bottom (13) connected to the equatorial edge (26) by a frustoconical wall (14).

14. A cotyloid component according to any one of claims 1 to 12, characterised in that the cup (3) has a cavity (32) for the spherical head (4) of the femoral component which extends over more than a hemisphere, the elasticity of the rim of the cup being sufficient to allow the passage of the spherical head (4).

FIG.1

FIG.2

EP 0 225 819 B1

FIG.3

FIG.4

FIG.5